# NEW EUROPEAN PATENT SPECIFICATION

(11) **EP 1 428 470 B2**
(45) Date of publication and mention of the opposition decision: **01.04.2020**
(45) Mention of the grant of the patent: 27.06.2012
(21) Application number: 04003911.7
(22) Date of filing: 18.05.2001
(51) Int. Cl.: A61B 3/10, A61B 3/11, A61F 9/013, A61F 9/01

(54) **Apparatus for determining and ablating a corneal tissue volume of a receiving cornea for corneal lamellar grafting transplantation**
Vorrichtung zur Bestimmung und Abtragung von Hornhautgewebevolumen in einer lamellierenden keratoplastik Behandlung
Dispositif permettant de déterminer et de procéder à l'ablation du volume de tissu cornéen requis en vue d'une greffe lamellaire de la cornée

(30) Priority: 15.09.2000 IT MI20002024
(43) Date of publication of application: 16.06.2004
(62) Divisional of application: 01936806.7
(73) Proprietor: IVIS TECHNOLOGIES S.r.l, 74100 Taranto (IT)
(72) Inventor: D'Ippolito, Giuseppe, 74100 Taranto (IT)
(74) Representative: Wegner, Hans

(56) References cited:
- EP-A- 0 247 260
- EP-A- 1 320 320
- EP-A1- 0 255 581
- EP-A1- 1 462 074
- WO-A1-02/22003
- DE-A1- 19 852 331
- US-A- 4 665 914
- US-A- 6 099 522
- US-B1- 6 551 306
- TERRY M A: "THE EVOLUTION OF LAMELLAR GRAFTING TECHNIQUES OVER TWENTY-FIVE YEARS" CORNEA, MASSON PUBL., NEW YORK, NY, US, vol. 19, no. 5, September 2000 (2000-09), pages 611-616, XP001021541 ISSN: 0277-3740
- RICH L F: "EXPANDING THE SCOPE OF LAMELLAR KERATOPLASTY" TRANSACTIONS OF THE AMERICAN OPHTHALMOLOGICAL SOCIETY ANNUAL MEETING, TORONTO, CA, vol. XCVII, 1999, pages 771-814, XP001021900 ISSN: 0065-9533
- ECKHARD H B ET AL: "LAMELLIERENDE KERATOPLASTIK MIT DEM EXCIMERLASER ERSTE KLINISCHE ERGEBNISSE", OPHTHALMOLOGE, SPRINGER, BERLIN, DE, vol. 93, no. 3, 1 June 1996 (1996-06-01), pages 242-246, XP001018490, ISSN: 0941-293X
- Program of ASCRS-ASOA "Symposium on Cataract, IOL and Refractive Surgery-Congress on Ophthalmic Practice Management - Clinical and Surgical Staff Program", May 20-24, 2000, Boston, Massachusetts, USA: Pages 33 and 130
- American Society of Cataract and Refractive Surgery "Abstracts - Symposium on Cataract, 1CL and Refractive Surgery", May 20-24, 2000, Boston, Massachusetts, USA: Page 43
- Buratto L. et al: "Excimer laser lamellar keratoplasty of augmented thickness for keratoconus", JOURNAL OF REFRACTIVE SURGERY, vol. 14, no. 5, 1 September 1998 (1998-09-01), pages 517-525, SLACK INC, US
- Kubota T. et al: "Lamellar excimer laser keratoplasty: reproducible photoablation of corneal tissue. A laboratory study", DOCUMENTA OPHTHALMOLOGICA, vol. 82, no. 3, 1 January 1992 (1992-01-01), pages 193-200, KLUWER, DORDRECHT, NL
- TERRY M.A.: "THE EVOLUTION OF LAMELLAR GRAFTING TECHNIQUES OVER TWENTY-FIVE YEARS", Cornea: the journal of cornea and external disease, vol. 19, no. 5, 1 September 2000 (2000-09-01), pages 611-616, US
- Böhnke M. et al: "Continuous non-contact corneal pachymetry with a high speed reflectometer", J. Refract. Surg., vol. 14, no. 2, March 1998 (1998-03), pages 140-146, University of Bern, Switzerland
- News, "Pachymetry Assisted Laser Keratoplasty (PALK), Page 1 of 4
- Milestones of the Company "Schwind eye-tech-solutions", Page 1 of 2
- RICH L F: "EXPANDING THE SCOPE OF LAMELLAR KERATOPLASTY UPA", American Ophthalmological Society. Transactions, vol. XCVII, 1 January 1999 (1999-01-01), pages 771-814, US ISSN: 0065-9533
- Print of an Internet page published by "Eye World News Magazine", entitled "ASCRS takes Beantown by storm", dated July 2000
- Print of an Internet page of the ASCRS entitled "New & Notewothy", retrieved with the help of the Web Archive Machine, dated 19 June 2000

## Description

### BACKGROUND OF THE INVENTION

The present invention relates to an apparatus for determining and ablating a corneal tissue volume of a receiving cornea for corneal lamellar grafting transplantation, as optimized for each individual patient.

As is known, in a lamellar corneal grafting operation, a portion of corneal tissue having an even thickness and a variable diameter, depending on the amount and location of the different specific pathologies, is conventionally removed.

Usually, the corneal tissue is removed by a surgical instrument called "Krumeich's microkeratome" which, by a planing type of operation removes a corneal disc having a preset diameter and an approximatively even thickness.

Use of excimer laser for lamellar treatments is described in RICH L F: "EXPANDING THE SCOPE OF LAMELLAR KERATOPLASTY" TRANSACTIONS OF THE AMERICAN OPHTALMOLOGICAL SOCIETY ANNUAL MEETING, TORONTO, CA, vol XCVII, 1999, pages 771-814.

### SUMMARY OF THE INVENTION

Thus, the aim of the present invention is to provide such an apparatus which is suitable to mutually coordinate an assembly of apparatus and which is specifically designed for defining, in an unique and optimum manner, the position, area and volume of the corneal tissue to be removed by a laser ablating operation, in order to optimally perform the ablating operation itself.

Within the scope of the above mentioned aim, a main object of the present invention is to provide such an apparatus which is very efficient in operation and which provides accurate values to allow the operator to perform optimally the ablating operation, i.e. in a manner optimized for each individual patient.

Yet another object of the present invention is to provide such an apparatus which, owing to its peculiar constructional features, is very reliable and safe in operation.

Yet another object of the present invention is to provide such an apparatus which can be easily made by using easily available elements and materials.

According to the present invention, there is an apparatus for determining and ablating a corneal tissue volume of a receiving cornea for lamellar corneal grafting transplantation as claimed in claim 1.

### BRIEF DESCRIPTION OF THE DRAWINGS

Further characteristics and advantages of the present invention will become more apparent hereinafter from the following disclosure of a preferred, though not exclusive, embodiment of an apparatus for determining and ablating an optimum volume of a corneal tissue as necessary for performing a lamellar corneal grafting operation, which is illustrated, by way of an indicative, but not limitative, example, in the accompanying drawings, where:
Figure 1 is a flow diagram illustrating the operating flows which can be performed by the apparatus according to the present invention;
Figure 2 is a cross-sectional view illustrating an optimum patient receiving bed;
Figure 2a is a further cross sectional view illustrating a typical section of a donor lens;
Figure 3 illustrates a typical section of the optimum receiving bed;
Figure 3a represents an optimum typical section of the donor lens;
Figure 4 illustrates a typical section of the optimum patient ablating contour, according to the present invention;
Figure 4a represents a typical section of the donor lens ablating contour; and
Figure 5 represents a block diagram of the ablating apparatus according to the present invention.

### DESCRIPTION OF THE PREFERRED EMBODIMENTS

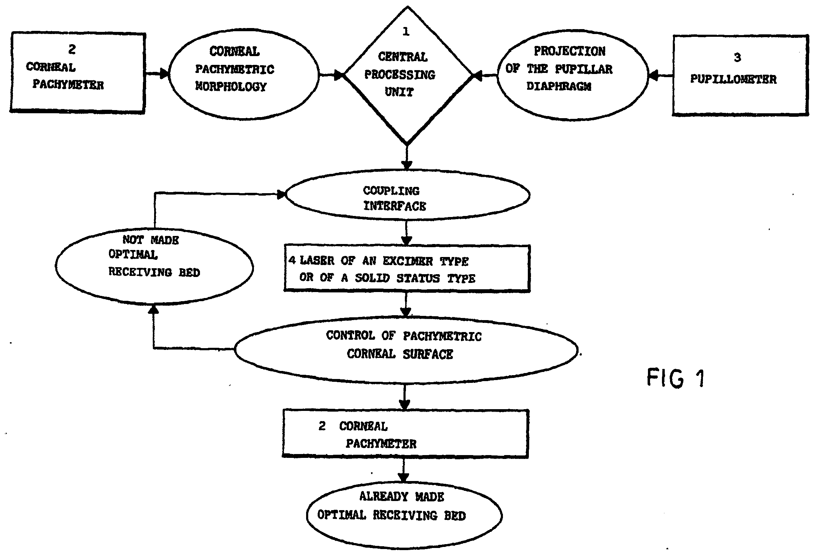

With reference to the number references of Figure 1, the apparatus for determining and removing, by an ablating operation, a corneal tissue volume necessary for a lamellar grafting transplantation, as optimized for the individual patient, comprises a central processing unit, generally indicated by the reference number 1, to which are coupled a corneal pachymeter (of an optical, ultrasound or the like type), generally indicated by the reference number 2, provided for morphologically detecting the corneal thickness and for tridimensionally mapping it.

To said central processing unit (1) a pupillometer, indicated by the reference number 3, for determining the projection of the pupillar diaphragm, at the level of the corneal front surface, is connected.

To said central processing unit 1 is moreover connected a photoablative laser (of an excimer, solid status, femtosecond or the like type), of a microspot type, generally indicated by the reference number 4, provided with a coupling interface, for reading the altimetric ablative datum, as expressed by microns on a x, y plane matrix or array.

The apparatus according to the present invention allows to find the ablating volume, which is obtained from a difference of the pachymetric map, as detected for the individual patient by the pachymeter 2, and the optimum pachymetric map of the bed receiving the donor lens.

The operator, in using the apparatus according to the invention, will detect at the start, by using the pupillometer 3, the projection of the pupil on the cornea front surface, the diameter thereof and the position of the related centroid with respect to the corneal limbus, as well as the diameter of the overall cornea and the position of its centroid, with respect to the pupillar centroid.

Then, the operator will detect the pachymetric data by using a corneal pachymeter (of an optical, ultrasonic or the like type) 2.

As is known, the pachymetric map is the tridimensional contour or profile which is best interpolatedly approximated to the data detected by the corneal pachymeter 2.

The optimum contour or profile of the donor lens receiving bed is defined by the operator by finding or detecting the following parameters:
- the center of the receiving bed;
- the diameter of the receiving bed;
- the minimum thickness at the center of the receiving bed
- the maximum thickness at the edge of the receiving bed; and
- the thickness variation along the diameter of the receiving bed.

The receiving bed center can be either selected between the corneal centroid, the projection on the cornea of the pupillar centroid, or arbitrarily by the operator.

The diameter of the receiving bed, in turn, is defined by the operator depending on the amount and location of the patient pathology.

The minimum and maximum thicknesses of the receiving bed are defined by the operator depending on the detected pathology analysis and the desired post-surgical refracting geometry.

The variation of the thickness along the receiving bed diameter is, depending on the operator selection, an even variation, a linear or exponential variation, depending on the desired post-surgical refracting geometry.

By using the definition of the above mentioned parameters, the operator will detect, in an unique and optimum manner for the patient, the receiving bed provided for receiving lens of the donor.

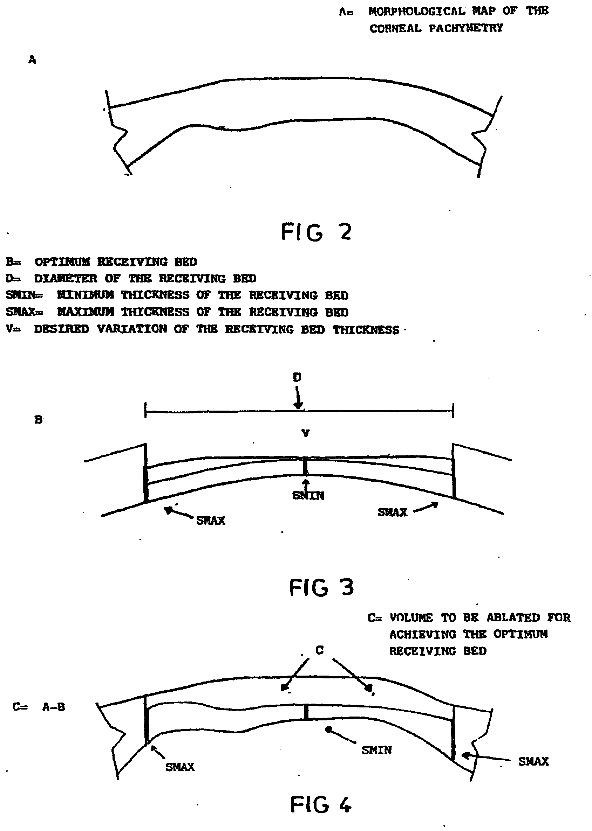

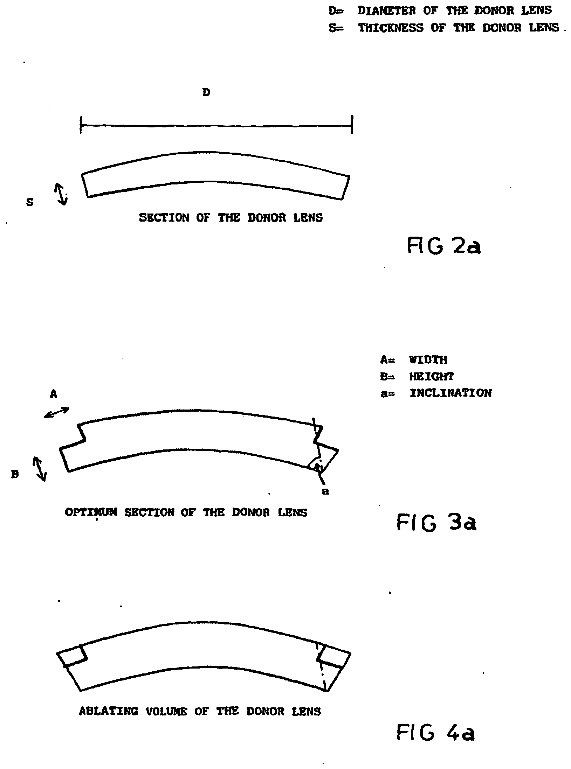

The ablating volume shown in Figure 2 is then obtained from the difference of the pachymetric map, as determined as above indicated, and the thus defined receiving bed.

The above disclosed method will univocally define the tissue to be ablated volume, in order to set an optimum patient receiving bed.

It is graphically represented by different color areas, clearly showing the receiving bed, its location and the residual cornea area not involved in the operation.

Moreover, digital data will express the overall ablating surface, the overall ablating volume, the maximum and minimum ablating volumes and related planimetric locations as well as the planimetric location of the ablating center with respect to the pupillar centroid.

The ablating volume related to the donor lens is obtained by defining the width A and height B of figure 3a to be generated on the lens or, alternately, the desired inclination or slope α.

The altimetric ablative datum is represented on a x, y-plane square matrix, to allow the photoablative laser 4 to properly perform its detection operation, through a suitable interface, for detecting the ablating contour or profile which, upon detection, is practically followed.

The system, moreover, optionally through an intra-operating detection of the pachymetric datum, will verify that the treatment has been carried out according to the programmed ablating strategy, while modifying the number of surface-unit localized pulses.

In this case, the operation will end upon achieving a congruency of the detected and desired data.

It should be pointed out that figure 3 clearly shows the optimum receiving bed B, the letter D showing the diameter of said receiving bed.

In this figure, Smin shows the minimum thickness of the receiving bed, whereas Smax shows the maximum thickness of said receiving bed.

The letter V shows, in turn, the desired variation of the receiving bed thickness.

In figure 4, the letter C shows the volume to be ablated for achieving the optimum receiving bed.

In this figure, moreover, Smax and Smin show respectively the maximum and minimum thickness of the mentioned receiving bed.

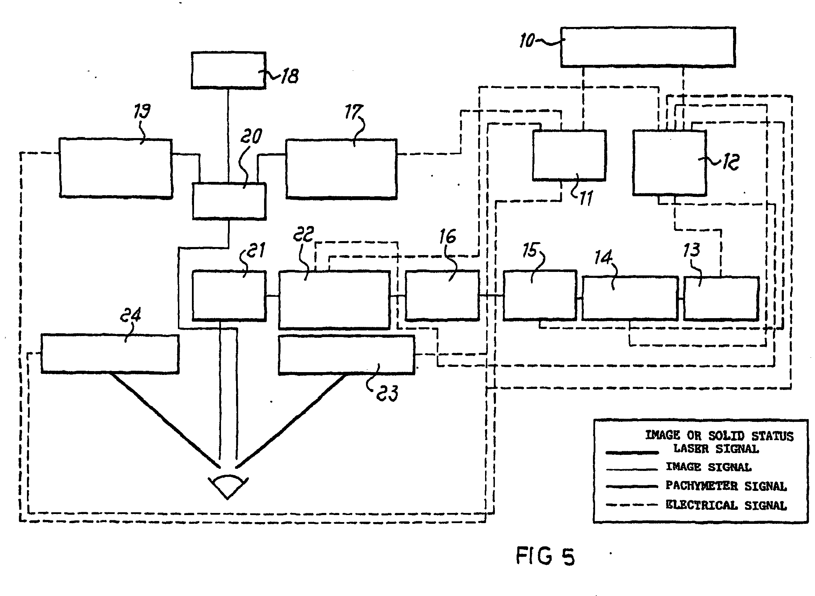

With reference to figure 5, which is a block diagram of the apparatus according to the invention, said apparatus comprises a central processing unit 10, which is coupled to a pachymetric control unit 11 and a photoablative laser control unit 12.

The latter is coupled to a laser cavity 13, fitted, in turn, to a control device for controlling the power of the laser beam 14 and to a device 15 for measuring the laser beam power.

This apparatus is moreover characterized in that it further comprises a focalizing system 16 for focalizing the laser beam, coupled to a galvanometric system 22 and to an orienting system 21 for orienting the laser beam.

Moreover, a system 23 for detecting the corneal pachymetry and a source 24 for detecting said corneal pachymetry are moreover provided.

The apparatus comprises furthermore an optic divider 20, to divide or split the video signal between an operating microscope 18, a video camera 19 for detecting the ocular motility, coupled to the photoablative laser control unit 12 and a further video camera 17 for detecting the ocular motility, coupled to the pachymeter control unit 11.

From the above disclosure it should be apparent that the invention fully achieves the intended aim and objects.

In particular, an apparatus according to the block diagram shown in figure 5 has been provided, which is adapted to allow to detect in a very accurate manner the data and/or parameters necessary for addressing and properly performing the surgical operation.

The invention, as disclosed, is susceptible to several modifications and variations, all of which will come within the scope of the invention defined in the appended claims.

Moreover, the used materials, as well as the contingent size and shapes, can be any, depending on requirements.

## Claims

1. An apparatus for determining and ablating a corneal tissue volume of a receiving cornea for corneal lamellar grafting transplantation,
comprising
a central processing unit (1, 10),
a pachymeter (2), coupled to the central processing unit (1, 10) for providing a corneal thickness tridimensional map (A), and
a photoablative laser (4);
**characterised in that**
the central processing unit (1, 10) comprises processing means for determining a volume (V) of corneal tissue to be ablated on the basis of a difference between the corneal thickness tridimensional map (A) and a receiving bed (B), having a predetermined bed thickness map, and
the photoablative laser (4) is controlled by the central processing unit (1, 10) for ablating said volume (V) of corneal tissue.

2. Apparatus according to claim 1, **characterised in that** it comprises a photoablative laser control unit (12), coupled to said said central processing unit (1, 10) for controlling a number of surface-unit localized pulses supplied by the photoablative laser (4) according to a predetermined ablating strategy and to intra-operating pachymetric detection.

## Patentansprüche

1. Vorrichtung zur Bestimmung und Abtragung von Hornhautgewebevolumen einer Empfängerhornhaut für eine lamelläre Hornhauttransplantation, die
eine zentrale Verarbeitungseinheit (1, 10),
ein Pachymeter (2), das mit der zentralen Verarbeitungseinheit (1, 10) gekoppelt ist, um eine dreidimensionale Abbildung (A) der Hornhautdicke bereitzustellen, und
einen photoablativen Laser (4) umfasst;
**dadurch gekennzeichnet, dass**
die zentrale Verarbeitungseinheit (1, 10) Verarbeitungsmittel zum Bestimmen eines Volumens (V) von abzutragendem Hornhautgewebe auf der Grundlage einer Differenz zwischen der dreidimensionalen Abbildung (A) der Hornhautdicke und einem Empfängerbett (B) mit einer vorbestimmten Bettdickenabbildung umfasst, und
der photoablative Laser (4) durch die zentrale Verarbeitungseinheit (1, 10) gesteuert wird, um dieses Hornhautgewebevolumen (V) abzutragen.

2. Vorrichtung nach Anspruch 1, **dadurch gekennzeichnet, dass** sie eine Steuereinheit (12) für den photoablativen Laser umfasst, die mit der zentralen Verarbeitungseinheit (1, 10) gekoppelt ist, um eine Anzahl von je Oberflächeneinheit lokalisierten Pulsen zu steuern, die von dem photoablativen Laser (4) gemäß einer vorbestimmten Abtragungsstrategie und einer intraoperativen pachymetrischen Detektion bereitgestellt werden.

## Revendications

1. Un appareil pour la détermination et l'ablation d'un volume de tissu cornéen d'une cornée receveuse pour une transplantation par greffe lamellaire de cornée, comprenant :
une unité centrale de traitement (1, 10),
un pachymètre (2), couplé à l'unité centrale de traitement (1, 10) pour produire une carte tridimensionnelle d'épaisseur de cornée (A), et
un laser de photoablation (4);
**caractérisé en ce que**
l'unité centrale de traitement (1, 10) comprend des moyens de traitement pour la détermination d'un volume (V) de tissu cornéen à ablater sur la base d'une différence entre la carte tridimensionnelle d'épaisseur de cornée (A) et un lit receveur (B), ayant une carte prédéterminée d'épaisseur de lit (A), et
le laser de photoablation (4) est contrôlé par l'unité centrale de traitement (1, 10) pour ablater ledit volume (V) de tissu cornéen.

2. Appareil selon la revendication 1, **caractérisé en ce qu'**il comprend une unité de contrôle de laser de photoablation (12), couplée à ladite unité centrale de traitement (1, 10) pour contrôler un nombre d'impulsions localisées par unité de surface délivrées par le laser de photoablation (4) en fonction d'une stratégie prédéterminée d'ablation et d'une détection pachymétrique intra-opératoire.
